# EUROPEAN PATENT SPECIFICATION

(11) **EP 2 934 478 B1**
(45) Date of publication and mention of the grant of the patent: **28.03.2018**
(21) Application number: 13799471.1
(22) Date of filing: 22.11.2013
(51) Int. Cl.: A61K 8/895, A61K 8/81, A61Q 19/00

(54) **SILICON-CONTAINING POLYOLEFINS IN PERSONAL CARE APPLICATIONS**
SILIKONHLATIGE POLYOLEFINE IN KÖRPERPFLEGEMITTELN
POLYOLÉFINES CONTENANT DU SILICIUM DANS DES APPLICATIONS DE SOIN PERSONNEL

(30) Priority: 19.12.2012 US 201261739452 P
(43) Date of publication of application: 28.10.2015
(73) Proprietor: Dow Global Technologies LLC, Midland, MI 48674 (US); Rohm and Haas Company, Philadelphia, PA 19106 (US)
(72) Inventor: CLARK, Thomas P., Midland, MI 48640 (US); KALIHARI, Vivek, Missouri City TX 77459-1139 (US); KAMBER, Nahrain E., Midland MI 48674 (US); LU, Xiaodong, North Wales, PA 19454 (US); O'CONNOR, Ying, Coatesville, PA 19320 (US); PETERSON, Thomas H., Midland, MI 48640 (US); SCHWARTZ, Curtis, Ambler, PA 19002 (US); WAN, Qichun, Midland, MI 48640 (US)
(74) Representative: Houghton, Mark Phillip
(86) International application number: PCT/US2013/071403
(87) International publication number: WO 2014/099256

(56) References cited:
- US-A- 4 446 283
- US-A- 4 834 972
- US-A- 5 219 560

## Description

### Field of the Invention

The present invention relates to personal care formulations having improved sensory feel, while also being non-tacky and easily spreadable on skin. These improved personal care formulations contain silane functionalized polyolefins. The present invention also relates to methods for improving the sensory feel of personal care formulations by including silane functionalized polyolefins in the formulations, as well as for treating skin by applying the aforesaid formulations.

### Background of the Invention

Good sensory feel, i.e., softness and smoothness, is a necessity for both leave-on and rinse off types of skin care products, including face care products, body care products, hand care products, sunscreens, antiperspirants, deodorants, color cosmetics, and face/hand/body wash. Many sensory enhancers have been developed to address this need, such as cationic surfactants (which are cationic compounds with quaternary ammonium functional groups, also known as "cationic quats") and silicone oils (which are typically polysiloxanes with organic side chains). Cationic quats and silicone oils are commonly used in skin care formulations to improve their sensory feel characteristics. However, high levels of cationic quats cause irritation on skin and silicone oils don't provide sufficient sensory benefits to satisfy consumer needs.

Silicone elastomers were developed as alternatives to cationic quats and silicone oils and are currently considered one of the best sensory modifiers used in skin care formulations. Due to the unique structure of silicone elastomers (i.e., loose-crosslinked silicone polymer swollen in silicone oil) and large particle size, it has a skin feel unlike any of the silicone fluids or cationic quats. Their feel has been described as "velvety", "powdery", "smooth," and "cushion feel". Also, their skin feel can be modified by controlling the amount of solvent in the formula, and therefore the degree of swelling. In general, the irregular shapes of these soft elastomer particles give a distinctly different feel on the skin.

The major drawbacks of silicone elastomers, however, are high cost and limited compatibility with other solvents. As a result, silicone elastomers tend to be limited high end skincare products, with the aforesaid shortcomings prohibiting their broad application for the mass market. There is also a contemporary trend toward reducing the use of silicones due to unwanted build-up on treated surfaces and environmental persistence. The absence of technology for producing new materials and formulations with performance comparable to silicone oils limits entry of new personal care products into high-volume and high demand personal care markets.

International Patent Application Publication WO2011034836A1 describes a process for making melt-shaped articles, e.g., wire and cable components, by a process in 3ww which the starting materials for the stable thermoplastic composition comprising silane functionalized polyolefin polymers (in particular vinyltrimethoxysilane-grafted polyethylene (PE-g-VTMS)) are combined and reacted in situ during the melt-forming process, thereby avoiding the need for post-shaping external heat or moisture. These articles made of PE-g-VTMS containing thermoplastic compositions have greater compatibility between the silica-containing and polyolefin phases therein compared to other materials previously used to make such articles.

U.S. Patent Application Publication No. US2009/0214455A1 discloses a composition for coating keratin materials which is formed by applying two compounds, at least one of which is a silicone compound and which are capable of reacting with one another. The resulting compositions have better adhesion to keratin and better biocompatibility, i.e., improved feel and low odor.

JP 2008174571A discloses cosmetic compounds containing silicone-modified olefin waxes manufactured by addition reaction of olefin waxes with Si-H bond-containing silicones, in the presence of catalysts, wherein the olefin waxes comprise ethylene-diene copolymers and ethylene-C₃₋₁₂-olefin-diene copolymers. These cosmetics were shown to have good spreadability, water repellency, use feel, storage stability, and give softness, smoothness, emollient effect, and luster to skin and hair.

US Patent No. 7,863,361 discloses a silicone-based composition comprising combination of silicone polymer and alkyltrisiloxane for personal care applications, which provides a silicone composition with lower solids, while maintaining a desirable range of viscosity.

US 4834972 discloses products for the personal care industry formulated with gels comprising a telomer-copolymer of ethylene and a silane, and a solvent.

The present invention addresses the problem of providing personal care formulations having improved sensory feel, while retaining other desired characteristics including spreadability and non-tacky feel on skin, by using variants of the above-mentioned silicone-modified polyolefins. Such elastomeric polymers can be swelled or dissolved in the carrier fluids to produce formulations having a soft, silky, and smooth feel and are easily spreadable and non-tacky on skin. Such formulations can provide better cost-to-treat sensory performance and improved formulation compatibility, making them economically and technically viable skin care alternatives for mass market products.

### Summary of the Invention

The present invention provides a personal care formulation comprising: (A) at least one silane functionalized polyolefin; and (B) a carrier. The silane functionalized polyolefin (A) comprises polymerized units derived from: (i) 50 to 99.9 % by weight of polymerized units derived from one or more olefin monomers; and (ii) 0.1 to 50 % by weight of polymerized units derived from at least one polymerizable silane selected from the group consisting of vinyl trimethoxy silane, vinyl triethoxy silane, vinyl triacetoxy silane, gamma-(meth)acryloxy propyl trimethoxy silane and mixtures thereof, and wherein said at least one silane functionalized polyolefin (A) is crosslinked.

Furthermore, the weight percent is based on the total weight of the silane-containing polyolefin and the weight percents of components (a) and (b) totals 100%.

In some embodiments of the personal care formulation, the at least one silane functionalized polyolefin (A) comprises polymerized units derived from: (i) 97 to 99.5 % by weight of polymerized units derived from said least one C₂-C₄₀ olefin monomer; and (ii) 0.5 to 3 % by weight of polymerized units derived from said least one polymerizable silane.

In some embodiments of the personal care formulation, the silane functionalized polyolefin (A) comprises at least 50 % by weight of polymerized units derived from polyethylene, based on the total weight of the silane functionalized polyolefin (A).

In some embodiments of the personal care formulation, wherein the polymerizable silane is selected from the group consisting of vinyl trialkoxy silanes.

The carrier may be selected from the group consisting of: aromatic or aliphatic hydrocarbons, alcohols, aldehydes, ketones, amines, esters, oleochemically derived oils, ethers, glycols, glycol ethers, silicone oils, water, and combinations thereof. The personal care formulation may further comprise one or more other ingredients selected from emollients, thickeners, solvents, colorants and surfactants.

The present invention also provides a method for treating the body which comprises applying the aforesaid personal care formulation externally to the body.

The present invention also provides a method for improving the sensory feel of personal care formulations which comprises including in the formulation at least one silane functionalized polyolefin (A) as described above.

### Brief Description of the Drawings

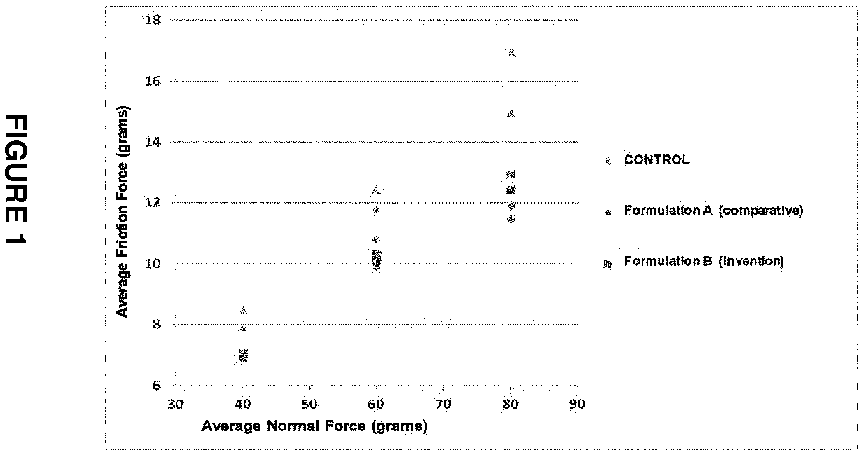

A more complete understanding of the present invention will be gained from the embodiments discussed hereinafter and with reference to the accompanying Figure which is a plot showing that skin care lotions of the present invention exhibit lower friction than the prior art at multiple normal loads, meaning that the skin care lotions of the present invention are less tacky and easier to spread.

### Detailed Description of the Invention

The present invention provides a personal care formulation comprising a silane functionalized polyolefin and a solvent. The silane functionalized polyolefin comprises polymerized units derived from one or more polyolefin monomers, such as one or more C₂-C₄₀ olefins, or one or more C₂-C₂₀ olefins, or one or more C₂-C₁₀ olefins, or preferably one or more C₂-C₈ olefins and at least one polymerizable silane. The silane units may have been copolymerized with the olefins or subsequently grafted onto the polyolefin polymer to form the silane functionalized polyolefin.

In some embodiments, the personal care formulation may further comprise one or more polyolefins different from the silane functionalized polyoelfins.

A method for improving the sensory feel of personal care formulations is also provided by the present invention and comprises including at least one silane functionalized polyolefin in the personal care formulation.

The present invention also provides a method for treating the body which comprises applying one or more of the above-described personal care formulations externally to the body.

The following terms, phrases and meanings are used hereinafter.

As used herein, "ambient conditions" and like terms means temperature, pressure and humidity of the surrounding area or environment of an article. The ambient conditions of a typical office building or laboratory include a temperature of 23 °C and atmospheric pressure.

Unless stated to the contrary, implicit from the context, or customary in the art, all parts and percents are based on weight and all test methods are current as of the filing date of this disclosure. For purposes of United States patent practice, the contents of any referenced patent, patent application or publication are incorporated by reference in their entirety (or its equivalent US version is so incorporated by reference) especially with respect to the disclosure of synthetic techniques, definitions (to the extent not inconsistent with any definitions specifically provided in this disclosure), and general knowledge in the art.

The numerical ranges in this disclosure are approximate, and thus may include values outside of the range unless otherwise indicated. Numerical ranges include all values from and including the lower and the upper values, in increments of one unit, provided that there is a separation of at least two units between any lower value and any higher value. As an example, if a compositional, physical or other property, such as, for example, molecular weight, viscosity, melt index, etc., is from 100 to 1,000, it is intended that all individual values, such as 100, 101, 102, etc., and sub ranges, such as 100 to 144, 155 to 170, 197 to 200, etc., are expressly enumerated. For ranges containing values which are less than one or containing fractional numbers greater than one (e.g., 1.1, 1.5, etc.), one unit is considered to be 0.0001, 0.001, 0.01 or 0.1, as appropriate. For ranges containing single digit numbers less than ten (e.g., 1 to 5), one unit is typically considered to be 0.1. These are only examples of what is specifically intended, and all possible combinations of numerical values between the lowest value and the highest value enumerated, are to be considered to be expressly stated in this disclosure. Numerical ranges are provided within this disclosure for, among other things, the component amounts of the composition and various process parameters.

"Polymer" means a compound prepared by reacting (i.e., polymerizing) monomers, as well as oligomers and other polymers, whether of the same or a different types. The generic term polymer thus embraces the term "homopolymer," usually employed to refer to polymers prepared from only one type of monomer, and the terms "copolymer" and "interpolymer" which are polymers prepared by the polymerization of two or more different types of monomers, oligomers and/or polymers. As will be readily recognized by persons of ordinary skill in the relevant art, oligomers are simply small polymers, i.e., having a smaller number (i.e., 2-100,000) of repeated monomer units.

"Olefins," also referred to herein as alkenes or alkene monomers, are unsaturated chemical compounds containing at least one carbon-to-carbon double bond, the simplest of which conform to the general formula CₙH₂ₙ, where n is a positive non-zero integer.

The phrase "comprising polymerized units derived from" as used hereinafter describes a polymer in terms of its constituent monomers. For example, a polymer comprising polymerized units derived from a polyolefin means that the polymer was formed from the polymerization reaction of at least one olefin monomer.

"Polyolefins" are polymers containing units derived from at least one type of olefin, typically a C₂-C₁₀ olefin, such as ethylene, propylene, butylene, pentene, hexane, etc. For example, polyethylene is a polymer which contains units derived from ethylene monomers, and typically comprises at least 50 mole percent (50 mol%) units derived from ethylene. Similarly, polypropylene contains units derived from propylene monomers, typically at least 50 mol% propylene.

"Blend," "polymer blend" and like terms mean a blend of two or more polymers. Such a blend may or may not be miscible. Such a blend may or may not be phase separated. Such a blend may or may not contain one or more domain configurations, as determined from transmission electron spectroscopy, light scattering, x-ray scattering, and any other method known in the art.

"Crosslinked" means that the polymer has been subjected or exposed to a treatment (e.g., heat, presence of free radicals, light, exposure to water, etc.) which induced reaction and bonding between functional groups of the polymer and other functional groups within the same polymer or the functional groups of other functionalized polymers. Furthermore, a polymer need not have all, or even most, of its functional groups reacted with other functional groups, to be considered a "crosslinked" polymer. In fact, a polymer having only a very small portion, such as at least 5 mole %, of the functional groups reacted or bonded is considered crosslinked, for purposes of the present invention.

"Crosslinkable" means that the polymer has not yet been crosslinked or bonded, but does comprise functional groups which will cause or promote crosslinking upon subjection or exposure to such treatment (e.g., exposure to water, heating, etc.).

"Composition," "formulation," and like terms, mean a physical mixture or blend of two or more components. For example, in the context of preparing silane functionalized polyethylene, a reaction composition, prior to reaction or polymerization, is a physical mixture which includes at least one ethylene polymer, at least one vinyl silane, and at least one free radical initiator. In the context of a personal care product, a typical formulation might include a solvent or carrier, and any desired additives such as lubricants, fillers, anti-oxidants, and the like.

The term "personal care formulation" as used hereinafter means a mixture or blend of compounds or ingredients which is suitable for external application to the body to deliver therapeutic or cosmetic compounds to skin and hair and may be in the form of creams, gels, lotions, liquids, sprays, powders, mousses and foams. Personal care formulations may be of the leave-on or rinse-off types, depending on their purpose and the types of compounds to be delivered. Specific products made from personal care formulations include, without limitation: deodorants, antiperspirants, and antiperspirant/deodorants; shaving creams or gels; skin lotions, moisturizers, and toners; bath and shower soaps, gels and lotions; cleansing products; hair care products such as shampoos, conditioners, mousses, styling gels, hair sprays, hair dyes, hair color products, hair bleaches, waving products, hair straighteners, hair frizz control, and hair volumizers; manicure products such as nail polish, nail polish remover, nail creams and lotions, cuticle softeners; protective creams and sprays such as sunscreen, insect repellent and anti-aging products, color cosmetics such as lipsticks, foundations, face powders, eye liners, eye shadows, blushes, makeup, mascaras, as delivery vehicles for fragrances, as well as drug delivery systems for topical application of medicinal compositions to the skin. More specific, but non-limiting examples of suitable personal care formulation ingredients include, for example, emollients, moisturizers, humectants, pigments, including pearlescent pigments such as, for example, bismuth oxychloride and titanium dioxide-coated mica, colorants, fragrances, biocides, preservatives, antioxidants, anti-microbial agents, anti-fungal agents, antiperspirant agents, exfoliants, hormones, enzymes, medicinal compounds, vitamins, salts, electrolytes, alcohols, polyols, ultraviolet radiation absorbing or blocking agents, botanical extracts, surfactants, silicone oils, organic oils, waxes, film formers, thickening agents such as, for example, fumed silica or hydrated silica, particulate fillers, such as for example, talc, kaolin, starch, modified starch, mica, nylon, clay, such as, for example, bentonite and organo-modified clays, and combinations thereof.

The personal care formulations comprising a silane functionalized polyolefin according to the present invention will now be described in detail. These personal care formulations have improved sensory feel, i.e., softness and smoothness, as well as the degrees of other characteristics desired by consumers, including spreadability, non-tackiness and absorption, compared to personal care formulations containing conventional sensory enhancers such as cationic quats and silicone oils.

### Silane Functionalized Polyolefins

A "silane functionalized polyolefin" means a polyolefin polymer having silane functionality. They may be random or block polymers, and linear or branched+. The silane functionality can be the result of either polymerizing one or more olefin monomers with at least one polymerizable silane, or grafting at least one polymerizable silane onto an existing polyolefin as described in further detail hereinafter. Grafting is typically understood by persons of ordinary skill in the art to mean that the additional monomer or functional group is bonded directly to the backbone of the existing polymer.

The recited silanes effectively copolymerize with one or more olefin monomers, or graft to, or crosslink with, a pre-existing polyolefin, to synthesize the silane functionalized polyolefin suitable for inclusion in the personal care formulation of the present invention.

Preferred silanes are the unsaturated alkoxy silanes can be grafted onto the polymer or copolymerized in-reactor with other monomers (such as ethylene and acrylates). These silanes and their method of preparation are more fully described in U.S. Pat. No. 5,266,627 to Meverden, et al. Vinyl trimethoxy silane (VTMS), vinyl triethoxy silane (VTES), vinyl triacetoxy silane, gamma-(meth)acryloxy propyl trimethoxy silane and mixtures of these silanes are the vinyl silanes for use in this invention.

The amount of polymerizable silane used in the practice of the present invention can vary widely depending upon the nature of the polymer, the silane, the processing or reactor conditions, the grafting or copolymerization efficiency, the ultimate application, and similar factors, but typically at least 0.1, preferably at least 0.5, weight percent is used. Considerations of convenience and economy are two of the principal limitations on the maximum amount of silane crosslinker used in the practice of this invention, and typically the maximum amount of silane crosslinker does not exceed 50, preferably it does not exceed 15, weight percent, based on the total amount of silane-containing polyolefin.

Olefin monomers suitable for polymerizing with at least one polymerizable silane to form the silane functionalized polyolefins useful in the present invention may be α-olefins, such as C₂₋₂₀, or C₂₋₁₀, or even C₂₋₈ α-olefins. Moreover, they may be linear, branched or cyclic α-olefins. Non-limiting examples of linear and branched α-olefins include ethylene, propene, 1-butene, 4-methyl-1-pentene, 1-hexene, 1-octene, 1-decene, 1-dodecene, 1-tetradecene, 1-hexadecene, and 1-octadecene. The α-olefins may also contain a cyclic structure such as cyclohexane or cyclopentane, resulting in an α-olefin such as 3-cyclohexyl-1-propene (allyl cyclohexane) and vinyl cyclohexane.

Although not α-olefins in the classical sense of the term, for purposes of this invention certain cyclic olefins, such as norbornene and related olefins, particularly 5-ethylidene-2-norbornene, are α-olefins suitable for inclusion in the polyolefins, which otherwise comprise at least 50 weight percent acyclic C₂₋₂₀ α-olefins. Similarly, styrene and its related olefins (for example, α-methylstyrene, etc.) are α-olefins for purposes of this invention.

The olefin monomers used to prepare the silane functionalized polyolefins may be of a single type, resulting in a silane functionalized homopolymer. On the other hand, more than one type of olefin monomer may be reacted with the polymerizable silane to form suitable silane functionalized polyolefins.

The silane functionalized polyolefin may be synthesized from olefin monomers and vinyl silanes using conventional polyolefin polymerization technology, such as high-pressure, Ziegler-Natta, metallocene, constrained geometry catalysis, among others well known to persons of ordinary skill in the relevant art. In some embodiments, polymerization may be performed using a high pressure process. Also in some embodiments, the silane functionalized polyolefin may be prepared using a mono- or bis-cyclopentadienyl, indenyl, or fluorenyl transition metal (preferably Group 4) catalysts or constrained geometry catalysts (CGC) in combination with an activator, in a solution, slurry, or gas phase polymerization process. For example, copolymerization of vinyl trialkoxy silanes with one or more olefin monomers may be done in a high-pressure reactor that is used in the manufacture of ethylene homopolymers and copolymers with vinyl acetate and acrylates.

Moreover, such polymerization may be accomplished at conditions well-known in the art, such as without limitation, at temperatures from 0 to 250 °C, preferably from 30 to 200 °C, and pressures from atmospheric to 10,000 atmospheres (1013 megaPascal (MPa)). Suspension, solution, slurry, gas phase, solid state powder polymerization or other process conditions may be employed, if desired. Where the polymerization reaction is catalyzed, the catalyst can be supported or unsupported, and the composition of the support can vary widely. Silica, alumina or a polymer (especially poly(tetrafluoroethylene) or a polyolefin) are representative supports, and desirably a support is employed when the catalyst is used in a gas phase polymerization process. The support is preferably employed in an amount sufficient to provide a weight ratio of catalyst (based on metal) to support within a range of from 1:100,000 to 1:10, more preferably from 1:50,000 to 1:20, and most preferably from 1:10,000 to 1:30. In most polymerization reactions, the molar ratio of catalyst to polymerizable compounds employed is from 10-12:1 to 10-1:1, more preferably from 10⁻⁹:1 to 10⁻⁵:1.

Inert liquids may serve as suitable solvents for polymerization. Examples include straight and branched-chain hydrocarbons such as isobutane, butane, pentane, hexane, heptane, octane, and mixtures thereof; cyclic and alicyclic hydrocarbons such as cyclohexane, cycloheptane, methylcyclohexane, methylcycloheptane, and mixtures thereof; perfluorinated hydrocarbons such as perfluorinated C₄₋₁₀ alkanes; and aromatic and alkyl-substituted aromatic compounds such as benzene, toluene, xylene, and ethylbenzene. Selection of a suitable solvent for polymerization is well within the ability of persons of ordinary skill in the relevant art.

As already noted, silane functionalized polyolefins suitable for inclusion in the personal care formulations of the present invention may be synthesized by grafting at least one vinyl silane onto an existing polyolefin polymer. The existing polyolefin polymers comprise polymerized units derived from one or more olefin monomers, such as one or more C₂₋₂₀ α-olefins, or one or more C₂₋₁₀ α-olefins, or even C₂₋₈ α-olefins. Moreover, the polyolefins may be homopolymers comprising only one type of olefin monomer, or they may be copolymers comprising two or more types of olefin monomers. The preferred class of vinyl silanes for grafting onto exiting polyolefins is the vinyl trialkoxy silanes.

Grafting the silane onto existing polyolefins may be accomplished by any conventional method, typically in the presence of a free radical initiator, e.g., peroxides and azo compounds, or by ionizing radiation, etc. Organic initiators are preferred, such as any one of the organic peroxide initiators, for example, dicumyl peroxide, di-tert-butyl peroxide, t-butyl perbenzoate, benzoyl peroxide, cumene hydroperoxide, t-butyl peroctoate, methyl ethyl ketone peroxide, 2,5-dimethyl-2,5-di(t-butyl peroxy)hexane, lauryl peroxide, and tert-butyl peracetate. A suitable azo compound is 2,2-azobisisobutyronitrile. The amount of initiator can vary, but it is typically present in an amount of at least 0.04, preferably at least 0.06, parts per hundred resin (phr). Typically, the initiator does not exceed 0.15, preferably it does not exceed about 0.10, phr. The weight ratio of silane crosslinker to initiator also can vary widely, but the typical crosslinker:initiator weight ratio is between 10:1 to 500:1, preferably between 18:1 and 250:1. As used in parts per hundred resin or phr, "resin" means the olefinic polymer.

While any conventional method can be used to graft the silane crosslinker to the polyolefin polymer, one particularly suitable method is blending the two with the initiator in the first stage of a reactor extruder, such as a Buss kneader. The grafting conditions can vary, but the melt temperatures are typically between 160 and 260 °C., preferably between 190 and 230 °C., depending upon the residence time and the half life of the initiator.

While various kinds of polyolefins may be used to synthesize grafted silane functionalized polyolefin suitable for the present invention, the present invention will now be described with polyethylene homo- or co-polymers as the polyolefin. As will be recognized by persons of ordinary skill in the relevant art, use of other types of polyolefins are equally acceptable for the present invention, and much of the following detailed discussion will be instructional and analogously applicable to other types of polyelofins when used for the present invention.

Clearly, ethylene homopolymers may be used with the aforesaid grafting process to synthesize silane functionalized polyolefins suitable for use in the present invention. Additionally, ethylene/α-olefin copolymers may be used having an ethylene content of at least 50 wt % and an α-olefin content of at least about 15, preferably at least about 20 and even more preferably at least about 25, wt %, based on the total weight of the copolymer. These copolymers typically have an α-olefin content of less than about 50, preferably less than about 45, more preferably less than about 40 and even more preferably less than about 35, wt % based on the total weight of the copolymer. The α-olefin content is measured by ¹³C nuclear magnetic resonance (NMR) spectroscopy using the procedure described in Randall (Rev. Macromol. Chem. Phys., C29 (2&3)).

Illustrative ethylene copolymers include ethylene/propylene, ethylene/butene, ethylene/1-hexene, ethylene/1-octene, ethylene/styrene, and the like. Illustrative terpolymers include ethylene/propylene/1-octene, ethylene/propylene/butene, ethylene/butene/1-octene, ethylene/propylene/diene monomer (EPDM) and ethylene/butene/styrene. The copolymers can be random or block.

The ethylene-based polymers used in the practice of this invention can be used alone or in combination with one or more other ethylene-based polymers, e.g., a blend of two or more ethylene polymers that differ from one another by monomer composition and content, catalytic method of preparation, etc. If the ethylene-based polymer is a blend of two or more ethylene polymers, then the ethylene polymer can be blended by any in-reactor or post-reactor process.

Examples of ethylene polymers made with high pressure processes include (but are not limited to) low density polyethylene (LDPE), ethylene silane reactor copolymer (such as SiLINK.RTM. made by The Dow Chemical Company), ethylene vinyl acetate copolymer (EVA), ethylene ethyl acrylate copolymer (EEA), and ethylene silane acrylate terpolymers.

Specific examples of ethylene polymers that can be grafted with silane functionality to produce suitable silane-containing polyethylene include, without limitation, very low density polyethylene (VLDPE) (e.g., FLEXOMER ethylene/1-hexene polyethylene made by The Dow Chemical Company), homogeneously branched, linear ethylene/.alpha.-olefin copolymers (e.g., TAFMER by Mitsui Petrochemicals Company Limited and EXACT by Exxon Chemical Company), homogeneously branched, substantially linear ethylene/.alpha.-olefin polymers (e.g., AFFINITY and ENGAGE polyethylene available from The Dow Chemical Company), and ethylene block copolymers (e.g., INFUSE polyethylene available from The Dow Chemical Company). The more preferred ethylene polymers are the homogeneously branched linear and substantially linear ethylene copolymers. The substantially linear ethylene copolymers are especially preferred, and are more fully described in U.S. Pat. Nos. 5,272,236, 5,278,272 and 5,986,028.

Methods for grafting a vinyl trialkoxy silane comonomer onto an ethylene polymer backbone are described, for example, in U.S. Patent. Nos. 3,646,155 and 6,048,935.

### Preparation of Personal Care Formulations

The resulting silane functionalized polyolefin may be combined with a carrier, and optional other ingredients, to produce a personal care formulation having soft, silky, and smooth feel and are easily spreadable and non-tacky on the skin. Suitable carriers include, for example without limitation, aromatic or aliphatic hydrocarbons, alcohols, aldehydes, ketones, amines, esters, oleochemically derived oils, i.e., sunflower oil, ethers, glycols, glycol ethers, or silicone oils, or water.

When combined with such carriers, the silane functionalized polyolefins can be swelled or dissolved in the carrier, or even made into dispersions in water. Products containing the resulting personal care formulations deliver a soft, silky, and smooth feel and are easily spreadable and non-tacky on the skin. It is expected that these products will provide better cost-to-treat sensory performance and improved formulation compatibility in comparison to currently used, but more expensive, silicone elastomers.

The personal care formulation of the present invention may be in the form of a powder, liquid, pellet, bead, oil gel, oil paste, or an aqueous dispersion. It may be combined with other skin care ingredients, such as emollients (hydrocarbon oils, esters, natural oils, silicones), waxes, sensory modifiers, rheology modifiers, humectants (glycerin, etc), sunscreen actives, natural ingredients, bio-actives, colorants, hard particles, emulsifiers, solubilizers, and surfactants.

Where the personal care formulation is in the form of an oil gel / paste, the amount of crosslinked silane-modified polyolefin included therein is typically 1 to 60, preferably 2 to 20 % by weight, based on the total weight of the personal care formulation. Where the personal care formulation is in the form of an emulsion (lotion or cream), the amount of crosslinked silane-modified polyolefin included therein is typically 0.1 to 60, preferably 1 to 40 % by weight, based on the total weight of the personal care formulation.

Where the personal care formulation is in the form of an aqueous product, the amount of crosslinked silane-modified polyolefin included therein is typically 0.1 to 90, preferably 0.5 to 15 % by weight, based on the total weight of the personal care formulation.

### EXAMPLES

### Key Terminology

PDMS = a silicone polymer, poly(dimethylsiloxane)
AFFINITY GA1950 = linear ethylene/1-octene polymer available from The Dow Chemical Company
ENGAGE 7447 = copolymer of ethylene and octene available from DuPont Dow Elastomer LLC
ENGAGE-g-VTES = copolymer of ethylene and octene grafted with vinyl triethoxy silane (VTES), available from DuPont Dow Elastomer LLC
MWn = molecular weight, number basis
MWw = molecular weight, weight basis

### Comparative Example 1 (Control) Made in situ:

### Polyolefin - no silicone and not crosslinked

In a 250 ml beaker was placed AFFINITY GA1950 (9 g) and ENGAGE 7447 (3 g). To the beaker was added 84g of Isohexadecane (Permethyl 101A) to produce a 12.5 wt% solution. The reactor was fitted with an overhead stirrer, a nitrogen inlet (slow purge). A thermo controlled hotplate was used to heat the stirred suspension to 120°C and mixed until all the solids were melted and a homogenous solution was achieved. Discontinued heating, continued stirring with air cooled to 75-80°C, added proportional amount into the lotion formulation during the emulsification (see Table I).

### Comparative Example 2

### Silicone-modified polyolefin oil, 2% PDMS cross-linked (w/solvent)

In a 1L kettle reactor was placed AFFINITY GA1950 (28.1 g) and ENGAGE-g-VTES (8.88 g). To the jar was added 322 mL of hexadecane to produce a 12.5 wt% solution. The reactor was fitted with an overhead stirrer, a nitrogen inlet (slow purge) and reflux condenser. A heating mantle was used to heat the stirred suspension to 180°C. After maintaining the homogeneous solution at 180°C for 1 hour, silanol-terminated PDMS (6.0 mL, 0.375 equiv relative to contained VTMS) was added via syringe. Stirring and heating was continued for a period of 90 minutes, after which the reactor contents were poured into a large jar and allowed to cool to ambient temperature producing a smooth gel.

### Example 1 - Present Invention

### Crosslinked silane-modified polyolefin oil via H₂O, no silicone

In a 1L kettle was placed AFFINITY GA1950 (28.1 g) and ENGAGE-g-VTES (8.88 g). To the jar was added 322 mL of hexadecane to produce a 12.5 wt% solution. The reactor was fitted with an overhead stirrer, a nitrogen inlet (slow purge) and reflux condenser. A heating mantle was used to heat the stirred suspension to 180°C. After maintaining the homogeneous solution at 180°C for 1h, H₂O (5 mL) was added via syringe. Stirring and heating was continued for a period of 90 minutes, after which the reactor contents were poured into a large jar and allowed to cool to ambient temperature to produce a smooth gel.

**Table I. Skin Lotion Formulation Ingredients (wt %)**

| Trade Name | INCI Name | Control | A | B |
|---|---|---|---|---|
| Phase I | | | | |
| DI Water | Water | q.s. to 100 | q.s. to 100 | q.s. to 100 |
| Glycerin | Glycerin | 2.00 | 2.00 | 2.00 |
| Keltrol CG-SFT (CP Kelco) | Xanthan Gum | 0.70 | 0.70 | 0.70 |
| | | | | |
| Phase II | | | | |
| Procol CS-20-D (Protameen) | Cetearyl Alcohol (and) Ceteareth 20 | 3.00 | 3.00 | 3.00 |
| RITA GMS (RITA) | Glyceryl Stearate | 2.00 | 2.00 | 2.00 |
| Permethyle 101A | Isohexadecane | 42.00 | | |
| Affinity GA 1950 | Ethene-1-Octene Copolymer | 4.50 | | |
| Engage 7447 EL | Ethylene Butene Copolymer | 1.50 | | |
| Comp. Ex. 2 | | | 48.00 | |
| Example 1 (invention) | | | | 48.00 |
| | | | | |
| Phase III | | | | |
| Neolone PE (Dow) | Phenoxyethaol, Methylisothiazolinone | 0.60 | 0.60 | 0.60 |
| Citric Acid (50% Solution) | Citric Acid | pH=5.5∼6.5 | pH=5.5∼6.5 | pH=5.5∼6.5 |

| | | | | |
|---|---|---|---|---|
| Control Formulation - polyolefin of Comparative Example 1 (no silane, no silicone and not crosslinked) Formulation A - polyolefin of Comparative Example 2 (crosslinked with 2% silicone) Formulation B - polyolefin of Example 1 (polyolefin grafted with silane and crosslinked with water) | | | | |

### Procedure of Skin Lotion Preparation

The following general procedure was followed to produce three skin care lotions, each containing the ingredients as listed in Table 1 above and one of the above-described polyolefins, Comparative Examples 1 and 2 and Example 1.

In a suitable size vessel, add in Phase I water, start mixing at moderator speed; sprinkle Xanthan Gum into water while mixing, mix until all hydrates and free of particles; add Glycerin to batch, heat to 75-80°C while mixing;

In a separate suitable size vessel, combine Phase II ingredients; for the control formulation, pre-mix Permethyl 101A, Affinity GA 1950 and Engage 7447 EL as indicated in Comparative Example I, before combining with other Phase II ingredients; heat Phase II mixture to 75-80°C with agitation until a uniform translucent solution is achieved;

At 75-80°C add Phase II into Phase I with mixing at moderate to fast speed; mix until a uniform emulsion is achieved; turn off heat, air cool the batch while mixing;

When temperature is below 35°C, add Phase III into batch, mix until uniform; adjust pH with Citric Acid (50% solution) to pH5.5-6.5.

### In-vitro sensory evaluation

The in-vitro sensory evaluation of above-described sample skin care lotions was done by friction analysis using an automated tribometer. The friction samples were made by drawing down thin films (∼10 grams per square meter) of the skin care lotion on black lenetta plastic sheets using an automated coater. The friction measurements were done on a tribometer, where a steel ball (3/8"diameter) is dragged over a coating at a fixed velocity (1 mm/sec) and constant normal load, and the lateral friction force is measured. Two measurements were performed for each normal load to conform the reproducibility. The normal force (40-80 gm) was specifically chosen to broadly cover the force a person would put on their skin while applying the lotion.

The Figure demonstrates the in-vitro sensory test performance of skin care lotions comprising: Crosslinked silane-modified polyolefin via H₂0 (Formulation B), Crosslinked silicone-modified polyolefin via PDMS (Formulation A), and Non-crosslinked polyolefin (Control). The normal force applied to the sample surface is plotted on the horizontal axis and the corresponding measured dynamic friction force is plotted on the vertical axis. The higher friction force corresponds to higher drag a person would feel while applying the lotion on their skin. One expects a higher drag force if a lotion is tacky and/or if a lotion is hard to spread. The dynamic friction force captures both these factors and hence, a non-tacky and easy to spread skin care lotion should exhibit lower friction forces.

The plot shows that the lotions containing crosslinked polyolefins (crosslinked either by water or functionalized silicone) exhibit lower friction force than the non-crosslinked polyolefin at multiple normal loads. The lower friction force indicates that the crosslinked polyolefin samples are less tacky, more smooth/less abrasive, and easier to spread than the non-crosslinked polyolefin lotion sample.

## Claims

1. A personal care formulation comprising:
(A) at least one silane functionalized polyolefin comprising polymerized units derived from:
(i) 50 to 99.9 % by weight of polymerized units derived from one or more olefin monomers; and
(ii) 0.1 to 50 % by weight of polymerized units derived from at least one polymerizable silane selected from the group consisting of vinyl trimethoxy silane, vinyl triethoxy silane, vinyl triacetoxy silane, gamma-(meth)acryloxy propyl trimethoxy silane and mixtures thereof;
and wherein the weight percent is based on the total weight of said silane-containing polyolefin and the weight percents of components (i) and (ii) totals 100%; and
(B) a carrier;
wherein said at least one silane functionalized polyolefin (A) is crosslinked.

2. The personal care formulation of Claim 1, wherein said at least one silane functionalized polyolefin (A) comprises polymerized units derived from:
(i) 97 to 99.5 % by weight of polymerized units derived from said one or more, C₂-C₄₀, olefin monomer; and
(ii) 0.5 to 3 % by weight of polymerized units derived from said at least one polymerizable silane.

3. The personal care formulation of Claim 1, wherein said one or more olefin monomers are C₂-C₄₀ olefin monomer.

4. The personal care formulation of Claim 1, wherein said silane functionalized polyolefin (A) comprises at least 50 % by weight of polymerized units derived from polyethylene, based on the total weight of the silane functionalized polyolefin (A).

5. The personal care formulation of Claim 4, wherein said polyolefin (A) comprises at least 90 % by weight of polyethylene.

6. The personal care formulation of Claim 1, wherein said carrier is selected from the group consisting of: aromatic or aliphatic hydrocarbons, alcohols, aldehydes, ketones, amines, esters, oleochemically derived oils, ethers, glycols, glycol ethers, silicone oils, water, and combinations thereof.

7. The personal care formulation of Claim 1, further comprising one or more other ingredients selected from emollients, thickeners, solvents, colorants and surfactants.

8. A method for treating the body which comprises applying the personal care formulation of Claim 1 externally to the body.

9. A method for improving the sensory feel of personal care formulations which comprises including in said formulation (A) at least one silane functionalized polyolefin which comprises polymerized units derived from:
(i) 50 to 99.9 % by weight of polymerized units derived from one or more olefin monomers; and
(ii) 0.1 to 50 % by weight of polymerized units derived from at least one polymerizable silane selected from the group consisting of vinyl trimethoxy silane, vinyl triethoxy silane, vinyl triacetoxy silane, gamma-(meth)acryloxy propyl trimethoxy silane and mixtures thereof;
and wherein the weight percent is based on the total weight of said silane-containing polyolefin and the weight percents of components (i) and (ii) totals 100%; and
(B) a carrier;
and wherein said at least one silane functionalized polyolefin (A) is crosslinked.

## Patentansprüche

1. Eine Körperpflegeformulierung, beinhaltend:
(A) mindestens ein silanfunktionalisiertes Polyolefin, das polymerisierte Einheiten beinhaltet, abgeleitet aus:
(i) 50 bis 99,9 Gew.-% polymerisierten Einheiten, abgeleitet aus einem oder mehreren Olefinmonomeren; und
(ii) 0,1 bis 50 Gew.-% polymerisierten Einheiten, abgeleitet aus mindestens einem polymerisierbaren Silan, ausgewählt aus der Gruppe, bestehend aus Vinyltrimethoxysilan, Vinyltriethoxysilan, Vinyltriacetoxysilan, gamma-(Meth)acryloxypropyltrimethoxysilan und Mischungen daraus;
und wobei das Gewichtsprozent auf dem Gesamtgewicht des Silan enthaltenden Polyolefins basiert und sich die Gewichtsprozente der Komponenten (i) und (ii) auf 100 % summieren; und
(B) einen Träger;
wobei das mindestens eine silanfunktionalisierte Polyolefin (A) vernetzt ist.

2. Körperpflegeformulierung gemäß Anspruch 1, wobei das mindestens eine silanfunktionalisierte Polyolefin (A) polymerisierte Einheiten beinhaltet, abgeleitet aus:
(i) 97 bis 99,5 Gew.-% polymerisierten Einheiten, abgeleitet aus mindestens einem C₂-C₄₀-Olefinmonomer; und
(ii) 0,5 bis 3 Gew.-% polymerisierte Einheiten, abgeleitet aus mindestens einem polymerisierbaren Silan.

3. Körperpflegeformulierung gemäß Anspruch 1, wobei das eine oder die mehreren Olefinmonomere C₂-C₄₀-Olefinmonomer sind.

4. Körperpflegeformulierung gemäß Anspruch 1, wobei das silanfunktionalisierte Polyolefin (A) mindestens 50 Gew.-% polymerisierte Einheiten, abgeleitet aus Polyethylen, bezogen auf das Gesamtgewicht des silanfunktionalisierten Polyolefins (A), beinhaltet.

5. Körperpflegeformulierung gemäß Anspruch 4, wobei das Polyolefin (A) mindestens 90 Gew.-% Polyethylen beinhaltet.

6. Körperpflegeformulierung gemäß Anspruch 1, wobei der Träger aus der Gruppe ausgewählt ist, die aus Folgendem besteht: aromatischen oder aliphatischen Kohlenwasserstoffen, Alkoholen, Aldehyden, Ketonen, Aminen, Estern, oleochemisch abgeleiteten Ölen, Ethern, Glycolen, Glycolethern, Silikonölen, Wasser und Kombinationen daraus.

7. Körperpflegeformulierung gemäß Anspruch 1, die ferner einen oder mehrere Inhaltsstoffe, ausgewählt aus Erweichungsmitteln, Verdickungsmitteln, Lösungsmitteln, Färbemitteln und Tensiden, beinhaltet.

8. Ein Verfahren zum Behandeln des Körpers, das das externe Auftragen der Körperpflegeformulierung gemäß Anspruch 1 auf den Körper beinhaltet.

9. Ein Verfahren zum Verbessern des sensorischen Anfühlens von Körperpflegeformulierungen, das das Einschließen von mindestens einem silanfunktionalisierten Polyolefin in der Formulierung (A), das polymerisierte Einheiten beinhaltet, beinhaltet, abgeleitet aus:
(i) 50 bis 99,9 Gew.-% polymerisierten Einheiten, abgeleitet aus einem oder mehreren Olefinmonomeren; und
(ii) 0,1 bis 50 Gew.-% polymerisierten Einheiten, abgeleitet aus mindestens einem polymerisierbaren Silan, ausgewählt aus der Gruppe, bestehend aus Vinyltrimethoxysilan, Vinyltriethoxysilan, Vinyltriacetoxysilan, gamma-(Meth)acryloxypropyltrimethoxysilan und Mischungen daraus;
und wobei das Gewichtsprozent auf dem Gesamtgewicht des Silan enthaltenden Polyolefins basiert und sich die Gewichtsprozente der Komponenten (i) und (ii) auf 100 % summieren; und
(B) einen Träger;
und wobei das mindestens eine silanfunktionalisierte Polyolefin (A) vernetzt ist.

## Revendications

1. Une formulation de soins personnels comprenant :
(A) au moins une polyoléfine à fonctionnalité silane comprenant des unités polymérisées dérivées de :
(i) 50 à 99,9 % en poids d'unités polymérisées dérivées d'un ou de plusieurs monomères oléfiniques ; et
(ii) 0,1 à 50 % en poids d'unités polymérisées dérivées d'au moins un silane polymérisable sélectionné dans le groupe constitué de vinyltriméthoxysilane, de vinyltriéthoxysilane, de vinyltriacétoxysilane, de gamma-méthacryloxypropyltriméthoxysilane et de mélanges de ceux-ci ;
et le pourcentage en poids étant rapporté au poids total de ladite polyoléfine contenant un silane et les pourcentages en poids des constituants (i) et (ii) faisant un total de 100 % ; et
(B) un support ;
dans laquelle ladite au moins une polyoléfine à fonctionnalité silane (A) est réticulée.

2. La formulation de soins personnels de la revendication 1, dans laquelle ladite au moins une polyoléfine à fonctionnalité silane (A) comprend des unités polymérisées dérivées de :
(i) 97 à 99,5 % en poids d'unités polymérisées dérivées dudit au moins un monomère oléfinique en C₂ à C₄₀ ; et
(ii) 0,5 à 3 % en poids d'unités polymérisées dérivées dudit au moins un silane polymérisable.

3. La formulation de soins personnels de la revendication 1, dans laquelle lesdits un ou plusieurs monomères oléfiniques sont un monomère oléfinique en C₂ à C₄₀.

4. La formulation de soins personnels de la revendication 1, dans laquelle ladite polyoléfine à fonctionnalité silane (A) comprend au moins 50 % en poids d'unités polymérisées dérivées de polyéthylène, rapporté au poids total de la polyoléfine à fonctionnalité silane (A).

5. La formulation de soins personnels de la revendication 4, dans laquelle ladite polyoléfine (A) comprend au moins 90 % en poids de polyéthylène.

6. La formulation de soins personnels de la revendication 1, dans laquelle ledit support est sélectionné dans le groupe constitué : d'hydrocarbures aromatiques ou aliphatiques, d'alcools, d'aldéhydes, de cétones, d'amines, d'esters, d'huiles dérivées oléochimiquement, d'éthers, de glycols, d'éthers de glycol, d'huiles de silicone, d'eau, et de combinaisons de ceux-ci.

7. La formulation de soins personnels de la revendication 1, comprenant en outre un ou plusieurs autres ingrédients sélectionnés parmi des émollients, des épaississants, des solvants, des colorants et des tensioactifs.

8. Une méthode pour traiter le corps qui comprend l'application de la formulation de soins personnels de la revendication 1 de façon externe au corps.

9. Une méthode pour améliorer le toucher sensoriel de formulations de soins personnels qui comprend le fait d'inclure dans ladite formulation (A) au moins une polyoléfine à fonctionnalité silane qui comprend des unités polymérisées dérivées de :
(i) 50 à 99,9 % en poids d'unités polymérisées dérivées d'un ou de plusieurs monomères oléfiniques ; et
(ii) 0,1 à 50 % en poids d'unités polymérisées dérivées d'au moins un silane polymérisable sélectionné dans le groupe constitué de vinyltriméthoxysilane, de vinyltriéthoxysilane, de vinyltriacétoxysilane, de gamma-(méth)acryloxy propyl triméthoxysilane et de mélanges de ceux-ci ;
et le pourcentage en poids étant rapporté au poids total de ladite polyoléfine contenant un silane et les pourcentages en poids des constituants (i) et (ii) font un total de 100 % ; et
(B) un support ;
et dans laquelle ladite au moins une polyoléfine à fonctionnalité silane (A) est réticulée.
